# FASCICULE DE BREVET EUROPEEN

(11) **EP 2 152 680 B1**
(45) Date de publication et mention de la délivrance du brevet: **24.06.2015**
(21) Numéro de dépôt: 08805594.2
(22) Date de dépôt: 22.05.2008
(51) Int. Cl.: C07D 269/02, C07D 269/00, C07C 235/12

(54) **PROCEDE DE PREPARATION DE N-CARBOXYANHYDRIDE ENANTIOMERIQUEMENT ENRICHI**
VERFAHREN ZUR HERSTELLUNG VON ENANTIOMERISCH ANGEREICHERTEM N-CARBOXYANHYDRID
METHOD FOR PREPARING ENANTIOMERICALLY ENRICHED N-CARBOXYANHYDRIDE

(30) Priorité: 25.05.2007 FR 0703702
(43) Date de publication de la demande: 17.02.2010
(73) Titulaire: SANOFI, 75008 Paris (FR)
(72) Inventeur: BIGOT, Antony, F-75013 PariS (FR); LAMPILAS, Maxime, F-75013 PariS (FR)
(74) Mandataire: Senninger, Thierry
(86) Numéro de dépôt international: PCT/FR2008/000703
(87) Numéro de publication internationale: WO 2009/004141

(56) Documents cités:
- SAVRDA J ET AL: "activation of N,N-bis(alkoxycarbonyl) amino acids. SYNTHESIS OF N-ALKOXYCARBONYL AMINO ACID N-CARBOXYANHYDRIDES AND N,N-DIALKOXYCARBONYL AMINO ACID FLUORIDES, AND THE BEHAVIOUR OF THESE AMINO ACID DERIVATIVES" TETRAHEDRON, ELSEVIER SCIENCE PUBLISHERS, AMSTERDAM, NL, vol. 50, no. 18, 1994, pages 5309-5322, XP002287687 ISSN: 0040-4020
- WESSELY F: "Untersuchungen ueber Alpha-Amino-N-Carbonsaeureanhydride. I" HOPPE-SEYLER'S ZEITSCHRIFT FUER PHYSIOLOGISCHE CHEMIE, WALTER DE GRUYTER, BERLIN, DE, vol. 146, 1925, pages 72-90, XP009094274 ISSN: 0018-4888
- JIDONG ZHU AND CO: "Total synthesis of Microsclerodermin E" ANGEW. CHEM. INT. ED., vol. 42, 2003, pages 5348-5351, XP002465215
- COSTE JACQUES ET AL: "Coupling N-methylated amino acids using PyBroP-1 and PyCloP halogenophosphonium salts: Mechanism and fields of application" JOURNAL OF ORGANIC CHEMISTRY, AMERICAN CHEMICAL SOCIETY. EASTON, US, vol. 59, no. 9, 1994, pages 2437-2466, XP002259799 ISSN: 0022-3263
- AKSSIRA M ET AL: "New routes to 1,4-benzodiazepin-2,5-diones" TETRAHEDRON, ELSEVIER SCIENCE PUBLISHERS, AMSTERDAM, NL, vol. 50, no. 30, 1994, pages 9051-9060, XP003009450 ISSN: 0040-4020 cité dans la demande
- HANBY W E ET AL: "Synthetic Polypeptides" JOURNAL OF THE CHEMICAL SOCIETY, CHEMICAL SOCIETY. LETCHWORTH, GB, 1950, pages 3009-3013, XP009094366 ISSN: 0368-1769 cité dans la demande

## Description

La présente demande est relative à un procédé de préparation d'un *N*-carboxyanhydride énantiomériquement enrichi d'un alpha acide aminé, plus particulièrement du carboxyanhydride de la *(L)*- ou de la *(D)*-*N*-méthyl alanine. La demande est aussi relative à un intermédiaire chimique qui est utilisé dans la préparation du carboxyanhydride de la *(L)-*ou de la *(D)*-*N*-méthyl alanine.

### [Le domaine technique et le problème technique]

Les *N*-carboxyanhydrides d'acides d'aminés sont des agents acylants qui présentent l'avantage de ne pas former de sous-produits gênants dans les réactions d'acylation. Lorsqu'ils sont énantiomériquement enrichis, ils permettent de plus d'introduire un carbone chiral. Il s'agit donc de composés chimiques utiles dans les synthèses organiques, en particulier dans le cas des synthèses de composés pharmaceutiques qui sont souvent des synthèses à plusieurs étapes réactionnelles. Cependant, il est nécessaire que ces composés présentent une pureté suffisante, en particulier une pureté énantiomérique. Le procédé industriel de préparation doit être de plus simple et présenter un bon rendement global.

La Demanderesse a mis au point un procédé simple de préparation de *N*-carboxyanhydride d'alpha acide aminé présentant un bon rendement global et permettant d'accéder à un produits pur et énantiomériquement enrichi. Ce procédé s'applique plus particulièrement au carboxyanhydride de la *(L)-* ou de la *(D)*-*N*-méthyl alanine.

### [L'art antérieur]

Dans Tetrahedron 1994, 50, N°18, 5309-5322, la préparation de *N*-carboxyanhydrides protégés sur l'atome d'azote par RO-C(=O)- à partir de *N*,*N*-bis-alcoxycarbonyl)amino acides et du réactif de Vilsmeier-Haack SOCl₂/DMF.

Dans l'article « Untersuchungen über Alpha-amino-N-carbonäureanhydride. I » de Zeitschrift für Physiologische Chemie », Walter de Bruyter, Berlin 1925, 46, 72-90, la préparation de N-carboxyanhydrides est décrite mais sans aucune étape de purification.

Dans Angew. Chem. Int. Ed. 2003, 42, 5348-5351, la préparation de *N*-carboxyanhydrides protégés sur l'atome d'azote par RO-C(=O)- est décrite sur le Schéma 3 faisant référence à Tetrahedron Letters 1996, 37, 8439 mais aucune étape de purification n'est décrite.

Dans J. Org. Chem. 1994, 59, 2437-2446, la préparation de *N*-carboxyanhydrides est décrite mais utilisant une autre réaction chimique.

Dans Tetrahedron 1994, 50, 30, 9051-9060, la préparation de *N*-carboxyanhydrides à partir d'acides aminés protégés par tBuO-C(=O)- (BOC) et de PCl₃ est décrite. L'emploi de PCl₃ conduit à la formation de sous-produits phosphorés qui restent combinés au *N-*carboxyanhydride et qu'il est nécessaire d'éliminer par une étape de lavage avec un solvant perchloré non acceptable industriellement (CCl₄).

Dans Journal of Chemical Society **1950,** 3009-3013, la préparation de *N*-carboxyanhydrides est conduite à partir d'un mélange des deux acides aminés (D et L) protégés par MeO-C(=O)- et de SOCl₂ et non à partir de l'acide aminé D ou bien L. De plus, le produit final est obtenu après une étape de précipitation, une étape de recristallisation et une étape de sublimation. Le rendement pondéral calculé est de 39% uniquement.

Dans J. Mar. Chim. Heterocycl. 2002, 1, 44-47, la préparation de N-carboxyanhydrides est conduite à partir d'acide aminé protégé et de POCl₃.

Aucun de ces documents ne décrit ni ne suggère le procédé de l'invention.

### [Description de l'invention]

### définitions utilisées

- groupe alkyle : un groupe hydrocarboné aliphatique saturé linéaire ou ramifié. A titre d'exemples, on peut citer les groupes méthyle, éthyle, propyle, isopropyle, butyle, iso-butyle, tertio-butyle. Il s'agit de préférence d'un groupe (C₁-C₄) ;
- groupe alkényle : un groupe alkyle comprenant une double liaison C=C ;
- groupe cycloalkyle : un groupe alkyle cyclique comprenant entre 3 et 8 atomes de carbone, tous les atomes de carbone étant engagés dans la structure cyclique. A titre d'exemples, on peut citer les groupes cyclopropyle, cyclobutyle, cyclopentyle, cyclohexyle ;
- groupe aryle : un groupe aromatique comprenant de 6 à 10 chaînons, par exemple un groupe phényle.

La présente invention est relative à un procédé de préparation d'un *N*-carboxyanhydride énantiomériquement enrichi d'un alpha acide aminé de formule (IIIa) : 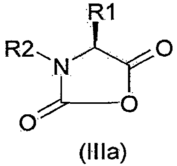 à partir du composé de formule (IIa) : 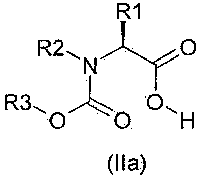 formules dans lesquelles :
- R1 et R2 désignent, indépendamment l'un de l'autre, un groupe alkyle (par ex. méthyle, éthyle, isopropyle),
- R3 désigne un groupe alkyle (par ex. méthyle, éthyle, tertio-butyle) ou -alkyle-aryle (par ex. benzyle) ;
comprenant les étapes suivantes :
(i) mettre en contact le composé de formule (IIa) (ou respectivement (IIb)) avec SOCl₂ dans un solvant;
(ii) précipiter le *N*-carboxyanhydride formé à l'étape (i) à l'aide d'un non-solvant ;
(iii) récupérer le *N*-carboxyanhydride.

Ce procédé ne comprend aucune étape de recristallisation, ni de sublimation du *N-*carboxyanhydride.

Ce procédé s'applique de façon similaire au composé (IIIb) à partir du composé (IIb) :

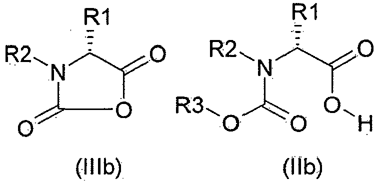

De préférence, R3 désigne un groupe méthyle ou éthyle, encore plus préférentiellement méthyle car à l'issue de l'étape (i), il se forme alors un composé léger (R3Cl) qui peut être éliminé facilement.

De préférence, R1 et R2 désignent, indépendamment l'un de l'autre, un groupe méthyle ou éthyle. Plus préférentiellement, R1 et R2 désignent tous deux un groupe méthyle et l'on obtient le *N*-carboxyanhydride de formule (A) (ou (B)) suivant : 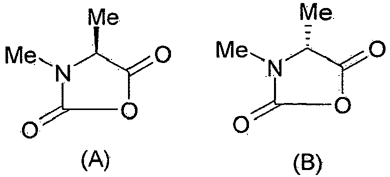
(A) est le *N*-carboxyanhydride de la (*L*)-*N*-méthyl-alanine. On utilise de préférence le composé (IIa) de formule : 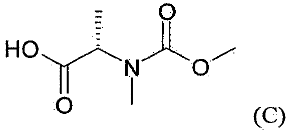 qui est le carbamate de méthyle de la (*L*)-*N*-méthyl alanine.
(B) le *N*-carboxyanhydride de la (*D*)-*N*-méthyl-alanine. On utilise de préférence le composé (IIb) qui est le carbamate de la (D)-N-méthyl alanine :

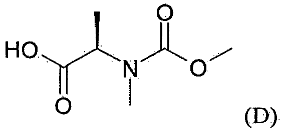

### Etape (i)

Le solvant de l'étape (i) peut-être un solvant chloré tel que le dichlorométhane, un éther d'alkyle ou d'aryle tel que par exemple le tétrahydrofurane, le 2-méthyl tétrahydrofurane, le 1,4-dioxanne, un solvant aromatique tel que par exemple le toluène, un xylène, le trifluorométhylbenzène, une cétone telle que par exemple l'acétone, la méthylisobutyl cétone, la méthyléthyl cétone. On peut utiliser également un mélange de deux ou plusieurs de ces solvants.

On utilise généralement entre 1 et 3 équivalent(s) de SOCl₂ par rapport au composé (IIa) (ou (IIb)).

La réaction est conduite à une température comprise entre -10 et 50°C, de préférence entre 0 et 30°C, encore plus préférentiellement entre 20 et 30°C. La durée de la réaction est comprise généralement entre 30 et 60 min.

### Etape (ii)

La précipitation du *N*-carboxyanhydride formé à l'étape (i) est conduite à l'aide d'un non-solvant. On utilise avantageusement un alcane liquide (*n*-heptane ou octane par ex.) ou bien une coupe pétrolière (éther de pétrole par ex.). On peut au préalable concentrer le *N-*carboxyanhydride en éliminant une partie du solvant de la réaction de l'étape (i).

On peut utiliser comme couple solvant/non-solvant, un solvant chloré et un alcane liquide ou une coupe pétrolière, notamment le couple dichlorométhane/n-heptane décrit dans l'exemple 2.

### Etape (iii)

Le *N*-carboxyanhydride peut être récupéré simplement par filtration/séchage. C'est là l'un des avantages du procédé de l'invention de ne pas nécessiter d'étape de recristallisation ou de sublimation pour obtenir une pureté suffisante et un bon rendement. L'avantage à utiliser un alcane liquide à l'étape (ii) est de pouvoir sécher facilement le *N*-carboxyanhydride.

### Préparation du composé de formule (IIa) ou (IIb)

Le composé de formule (IIa) (ou (IIb)) peut être préparé selon la réaction suivante (éventuellement au cours d'une étape précédant l'étape (i)): 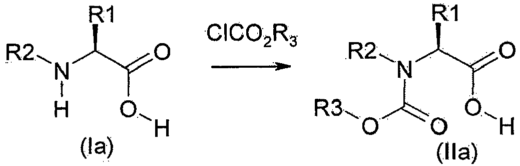 (ou bien respectivement selon 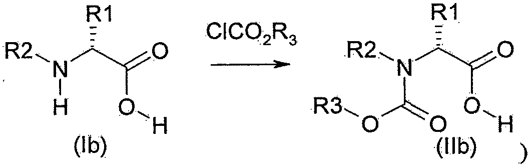 en présence d'une base. La base peut être par exemple un carbonate, un bicarbonate ou un hydroxyde d'un métal alcalin. Une base préférée est NaOH.

Cette réaction peut être conduite dans un solvant qui peut être par exemple l'eau ou l'un des solvants cités précédemment. La réaction est conduite à une température comprise entre 0 et 5°C. La durée de la réaction est comprise généralement entre 5 et 6 heures. De préférence, on utilise un rapport molaire ClCO₂R3/(IIa) (ou (IIb) > 1,9. De préférence aussi, on utilise un rapport molaire base/(IIa) (ou (IIb) > 2.

Le procédé de la présente invention permet d'obtenir un *N*-carboxyanhydride de façon simple, avec un bon rendement (> au rendement divulgué dans Journal of Chemical Society 1950) et une bonne pureté. En particulier, il ne nécessite aucune étape de recristallisation ni de sublimation. La cyclisation de l'étape (i) préserve l'intégrité du centre asymétrique et permet d'obtenir un produit énantiomériquement enrichi (aucune épimérisation).

### [Exemples]

L'homme du métier pourra s'inspirer avantageusement des conditions divulguées dans les deux exemples qui suivent.

### Ex 1 : Préparation de l'acide (S)-2-(méthoxycarbonyl-méthyl-amino)-propionique)

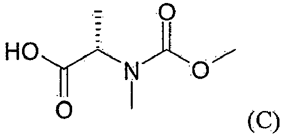

Dans un bicol de 250 ml sous atmosphère d'azote sont chargés successivement : 10 gr (97 mmoles) de (L)-*N*-méthyl alanine, puis 200 ml d'une solution aqueuse 1 M de NaOH (200 mmoles, 2,06 éq.). La suspension blanche ainsi obtenue est agitée jusqu'à complète dissolution (environ 30 minutes). Cette solution est refroidie à environ 3°C à l'aide d'un bain eau/glace, et sous une vigoureuse agitation (environ 750 tours/min), 15 ml de chloroformiate de méthyle (192 mmoles, 1,98 éq.) sont ajoutés en 30 minutes environ à l'aide d'une ampoule de coulée de 50 ml. Le milieu biphasique ainsi obtenu est agité à 3°C.

Au bout de 6 heures, la température du milieu réactionnel est remontée à environ 20°C en retirant le bain glace/eau, et le pH du milieu réactionnel est amené à environ 1 (mesuré à l'aide d'un papier pH) à l'aide d'HCl aqueux à 37%. Cette phase aqueuse est alors extraite par 3 fois 50 ml d'AcOEt. Les phases organiques sont réunies, lavées par 50 ml d'eau déminéralisée, puis séchées sur environ 10 gr de MgSO₄ anhydre. Le milieu est filtré sur verre fritté, puis le filtrat est concentré à sec sous vide (température du bain à environ 35° C, vide d'environ 40 mbars). On obtient ainsi 13,4 gr (86%) de produit sous forme d'une huile visqueuse incolore.

analyses structurales : LC-MS-DAD-ELSD : 160(-)=(M-H)(-), 162(+)=(M+H)(+) ; RMN, ¹H (DMSO d⁶ à 400 MHz) : pour ce lot, un mélange 60-40% de conformères est observé, avec : 1,31 (*d,* J = 7,5 Hz, 3H) ; 2,78 (s, 3H) ; 3,58 (s, 1,2H) ; 3,60 (s, 1,8H) ; 4,50 (*q*, J = 7,5 Hz, 0,4H) ; 4,58 (*q*, J = 7,5 Hz, 0,6H) ; 10,7 *(m étalé,* 1H).

Une analyse par chromatographie gaz sur colonne chirale permet de montrer que l'excès énantiomérique du carbamate de méthyle de la *N*-méthyl alanine est >99%. Les conditions de l'analyse chromatographique sont données ci-après : colonne RT-Gammadex (30 m / 0,25 mm / 0,25 µm) ; isotherme 120°C (3 min), puis 5°C/min à 180°C, isotherme à 180°C (3 min) ; injection split 1:25 ; gaz vecteur hélium à 1,8 ml/min ; détection FID ; solution à 2 mg/ml dans CH₂Cl₂, estérification avec TMSH 0,2 M. Dans ces conditions, le carbamate de méthyle de la (*L*)-*N*-méthyl alanine a un temps de rétention tr = 8,8 min. L'autre énantiomère, le carbamate de méthyle de la (*D*)-*N*-méthyl alanine présente un temps de rétention tr = 8,5 min.

### Ex 2 : Préparation de (S)-3,4-diméthyl-1,3-oxazolidine-2,5-dione)

1 gr (6,2 mmoles) du produit obtenu précédemment est mis en solution dans 3 ml de CH₂Cl₂, agité pendant 5 minutes à environ 20°C, puis traité par SOCl₂ (500 µl, 1,1 éq.). Le milieu réactionnel est alors chauffé à environ 30° C, et le chauffage est maintenu pendant environ 30 min. Le milieu réactionnel est alors concentré à environ 1,5 volume, et toujours sous agitation, 10 ml de *n*-heptane sont ajoutés. La masse blanche ainsi obtenue est alors refroidie à environ -20° C, et agitée pendant 1 heure à cette température. La suspension est alors filtrée sur verre fritté, le solide est lavé par 3 fois 3 ml de n-heptane. Après séchage à l'air pendant 2 heures, le produit (700 mg, 87,4%) est obtenu sous forme d'aiguilles blanches,

analyses structurales : RMN ¹H (DMSO d⁶ à 400 MHz) : 1,39 (*d*, J = 7,5 Hz, 3H) ; 2,83 (*s*, 3H) ; 4,40 (*q*, J = 7,5 Hz, 1 H).

Une analyse par chromatographie gaz sur colonne chirale permet de montrer que l'excès énantiomérique de la (*S*)-3,4-diméthyl-1,3-oxazolidine-2,5-dione) est >99%, et qu'aucune épimérisation n'a eu lieu dans les conditions de cyclisation. Conditions de la chromatographie gaz : colonne RT-Gammadex (30 m / 0,25 mm / 0,25 µm) ; température initiale 180°C puis 5°C/min à 220°C, isotherme à 180°C pendant 5 min ; injection split 1:25 ; gaz vecteur hélium à 1,8 ml/min ; détection FID ; solution à 2 mg/ml dans CH₂Cl₂. Dans ces conditions, la (*S*)-3,4-diméthyl-1,3-oxazolidine-2,5-dione a un temps de rétention tr = 8,4 min. L'autre énantiomère, la (*R*)-3,4-diméthyl-1,3-oxazolidine-2,5-dione) a un temps de rétention tr = 8,7 min.

Le rendement global calculé à partir de la (L)-*N*-méthyl alanine de départ est donc de 86% x 87,4% soit 75%. Ce rendement est supérieur à celui obtenu dans Journal of Chemical Society 1950**.**

## Revendications

1. Procédé de préparation d'un *N*-carboxyanhydride énantiomériquement enrichi d'un alpha acide aminé de formule (IIIa) : 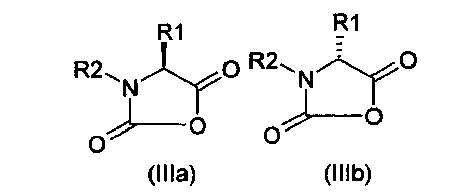 à partir du composé de formule (IIa) (ou respectivement (IIb) : 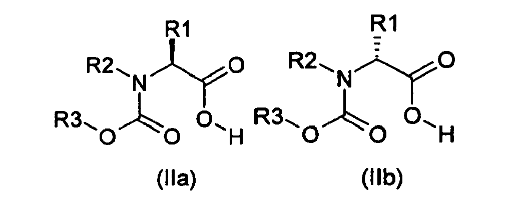 formules dans lesquelles :
• R1 et R2 désignent, indépendamment l'un de l'autre, un groupe alkyle ;
• R3 désigne un groupe alkyle ou alkyle-aryle ;
comprenant les étapes suivantes :
(i) mettre en contact le composé de formule (IIa) (ou respectivement (IIb)) avec SOCl₂ dans un solvant ;
(ii) précipiter le *N*-carboxyanhydride formé à l'étape (i) à l'aide d'un non-solvant ;
(iii) récupérer le *N*-carboxyanhydride.
et ne comprenant aucune étape de recristallisation, ni de sublimation du *N-*carboxyanhydride.

2. Procédé selon la revendication 1 dans lequel R3 désigne un groupe méthyle ou éthyle.

3. Procédé selon l'une des revendications 1 ou 2 dans lequel R1 et R2 désignent, indépendamment l'un de l'autre, un groupe méthyle ou éthyle.

4. Procédé selon la revendication 1 dans lequel R1, R2 et R3 désignent un groupe méthyle.

5. Procédé de préparation du *N*-carboxyanhydride énantiomériquement enrichi de formule (A) ou (B) : 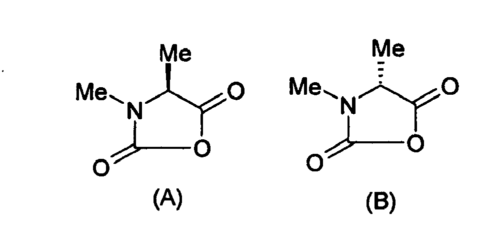 comprenant les étapes suivantes :
(i) mettre en contact le composé de formule 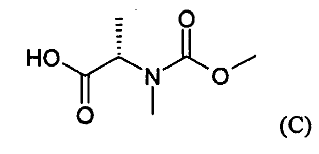 ou respectivement 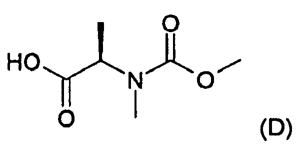 avec SOCl₂ dans un solvant;
(ii) précipiter le *N*-carboxyanhydride formé à l'étape (i) à l'aide d'un non-solvant ;
(iii) récupérer le *N*-carboxyanhydride ;
et ne comprenant aucune étape de recristallisation, ni de sublimation du *N-*carboxyanhydride.

6. Procédé selon l'une des revendications 1 à 5 dans lequel la réaction de l'étape (i) est conduite à une température comprise entre -10 et 50°C, de préférence entre 0 et 30°C, encore plus préférentiellement entre 20 et 30°C.

7. Procédé selon l'une des revendications 1 à 6 dans lequel la réaction de l'étape (i) est conduite avec de 1 à 3 équivalent(s) de SOCl₂ par rapport au composé (IIa) (ou respectivement (IIb)) ou (C) (ou respectivement (D))

8. Procédé selon l'une des revendications 1 à 7 dans lequel le non-solvant de l'étape (ii) est un alcane liquide ou une coupe pétrolière.

9. Procédé selon l'une des revendications 1 à 8 dans lequel on utilise comme couple solvant/non-solvant un solvant chloré et un alcane liquide ou une couple pétrolière, de préférence le couple dichlorométhane/n-heptane.

10. Procédé selon l'une quelconque des revendications 1 à 9 dans lequel la récupération du *N*-carboxyanhydride de l'étape (iii) est une filtration/séchage.

11. Composé de formule :

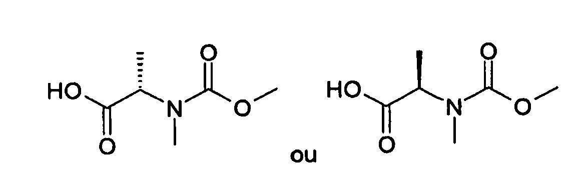

## Patentansprüche

1. Verfahren zur Herstellung eines enantiomerenangereicherten *N*-Carboxyanhydrids einer alpha-Aminosäure der Formel (IIIa): 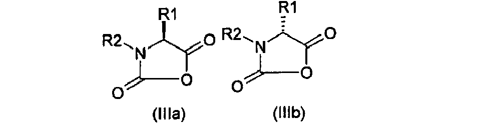 aus einer Verbindung der Formel (IIa) (bzw. (IIb)): 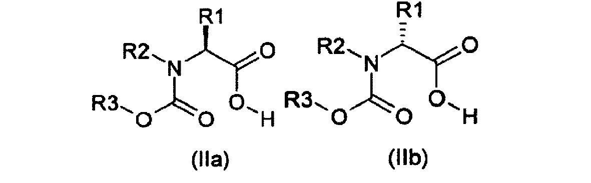 worin:
• R1 und R2 unabhängig voneinander für eine Alkylgruppe stehen;
• R3 für eine Alkyl- oder Alkylarylgruppe steht;
das folgende Schritte umfasst:
(i) Inberührungbringen der Verbindung der Formel (IIa) (bzw. (IIb)) mit SOCl₂ in einem Lösungsmittel;
(ii) Ausfällen des in Schritt (i) gebildeten *N*-Carboxyanhydrids mit Hilfe eines Nichtlösungsmittels;
(iii) Gewinnen des *N*-Carboxyanhydrids;
und das keinen Schritt der Umkristallisation oder Sublimation des *N*-Carboxyanhydrids umfasst.

2. Verfahren nach Anspruch 1, bei dem R3 für eine Methyl- oder Ethylgruppe steht.

3. Verfahren nach einem der Ansprüche 1 und 2, bei dem R1 und R2 unabhängig voneinander für eine Methyl- oder Ethylgruppe stehen.

4. Verfahren nach Anspruch 1, bei dem R1, R2 und R3 für eine Methylgruppe stehen.

5. Verfahren zur Herstellung von enantiomerenangereichertem *N*-Carboxyanhydrid der Formel (A) oder (B): 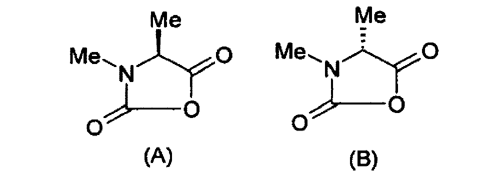 das folgende Schritte umfasst:
(i) Inberührungbringen der Verbindung der Formel 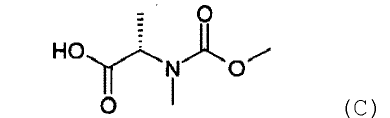 bzw. 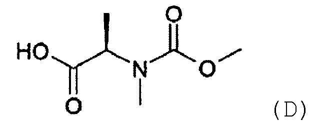 mit SOCl₂ in einem Lösungsmittel;
(ii) Ausfällen des in Schritt (i) gebildeten *N*-Carboxyanhydrids mit Hilfe eines Nichtlösungsmittels;
(iii) Gewinnen des *N*-Carboxyanhydrids;
und das keinen Schritt der Umkristallisation oder Sublimation des *N*-Carboxyanhydrids umfasst.

6. Verfahren nach einem der Ansprüche 1 bis 5, bei dem die Reaktion von Schritt (i) bei einer Temperatur zwischen -10 und 50°C, vorzugsweise zwischen 0 und 30°C und noch weiter bevorzugt zwischen 20 und 30°C durchgeführt wird.

7. Verfahren nach einem der Ansprüche 1 bis 6, bei dem die Reaktion von Schritt (i) mit 1 bis 3 Äquivalent(en) SOCl₂, bezogen auf die Verbindung (IIa) (bzw. (IIb)) bzw. (C) (bzw. (D)) durchgeführt wird.

8. Verfahren nach einem der Ansprüche 1 bis 7, bei dem es sich bei dem Nichtlösungsmittel von Schritt (ii) um ein flüssiges Alkan oder einen Erdölschnitt handelt.

9. Verfahren nach einem der Ansprüche 1 bis 8, bei dem man als Lösungsmittel/Nichtlösungsmittel-Paar ein chloriertes Lösungsmittel und ein flüssiges Alkan oder einen Erdölschnitt, vorzugsweise das Paar Dichlormethan/n-Heptan, verwendet.

10. Verfahren nach einem der Ansprüche 1 bis 9, bei dem es sich bei der Gewinnung des *N*-Carboxy-anhydrids von Schritt (iii) um eine Filtration/Trocknung handelt.

11. Verbindung der Formel:

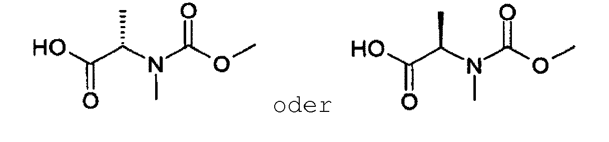

## Claims

1. Method for preparing an enantiomerically enriched α-amino acid *N*-carboxyanhydride of formula (IIIa): 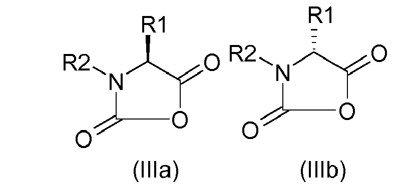 from the compound of formula (IIa) (or (IIb) respectively): 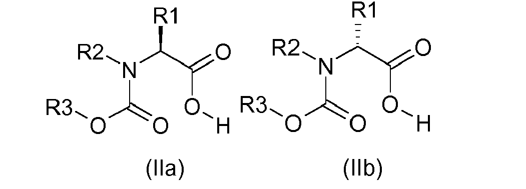 in which formulae:
• R1 and R2 denote, independently of one another, an alkyl group; and
• R3 denotes an alkyl or alkylaryl group;
comprising the following steps:
(i) bringing the compound of formula (IIa) (or (IIb) respectively) into contact with SOCl₂ in a solvent;
(ii) precipitating the *N*-carboxyanhydride formed in step (i) using a non-solvent; and
(iii) recovering the *N*-carboxyanhydride,
and not comprising any recrystallization step, nor a sublimation step of the *N*-carboxyanhydride.

2. Method according to Claim 1, in which R3 denotes a methyl or ethyl group.

3. Method according to either of Claims 1 and 2, in which R1 and R2 denote, independently of one another, a methyl or ethyl group.

4. Method according to Claim 1, in which R1, R2 and R3 denote a methyl group.

5. Method of preparing enantiomerically enriched *N-*carboxyanhydride of formula (A) or (B): 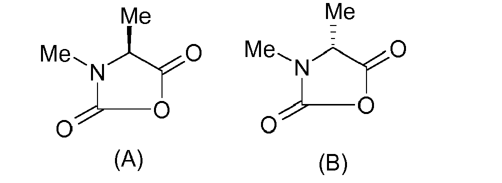 comprising the following steps:
(i) bringing the compound of formula 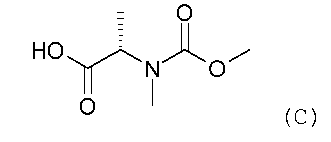 or respectively 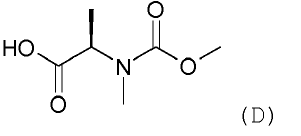 into contact with SOCl₂
in a solvent;
(ii) precipitating the *N*-carboxyanhydride formed in step (i) using a non-solvent; and
(iii) recovering the *N*-carboxyanhydride;
and not comprising any recrystallization step, nor a sublimation step of the *N*-carboxyanhydride.

6. Method according to one of Claims 1 to 5, in which the reaction from step (i) is carried out at a temperature between -10 and 50°C, preferably between 0 and 30°C, more preferably still between 20 and 30°C.

7. Method according to one of Claims 1 to 6, in which the reaction from step (i) is carried out with 1 to 3 equivalent(s) of SOCl₂ relative to the compound (IIa) (or (IIb) respectively) or (C) (or (D) respectively).

8. Method according to one of Claims 1 to 7, in which the non-solvent from step (ii) is a liquid alkane or an oil cut.

9. Method according to one of Claims 1 to 8, in which use is made, as a solvent/non-solvent pair, of a chlorinated solvent and a liquid alkane or oil cut, preferably the dichloromethane/n-heptane pair.

10. Method according to any one of Claims 1 to 9, in which the recovery of the *N*-carboxyanhydride from step (iii) is a filtration/drying operation.

11. Compound of formula:
